# EUROPEAN PATENT APPLICATION

(11) **EP 4 215 193 A1**
(43) Date of publication of application: **26.07.2023**
(21) Application number: 21869841.3
(22) Date of filing: 19.08.2021
(51) Int. Cl.: A61K 31/405, A61K 47/40, A61P 35/00

(54) **METHOD FOR THE CONSERVATIVE TREATMENT OF ENDOMETRIAL POLYPS**

(30) Priority: 16.09.2020 RU 2020130453
(71) Applicant: Kiselev, Vsevolod Ivanovich, Moscow 117218 (RU)
(72) Inventor: Kiselev, Vsevolod Ivanovich, Moscow 117218 (RU)
(74) Representative: Einsel, Martin
(86) International application number: PCT/RU2021/000357
(87) International publication number: WO 2022/060245

(57) **Abstract**

The invention relates to the field of medicine and can be used in the treatment of benign hyperplastic processes in the endometrium. The claimed method for treating endometrial polyps consists in administering to a female patient a drug comprising 3,3'-diindolyhnethane in the form of vaginal tablets which contain a complex of 3,3'-diindolylmethane with β-cyclodextrin in the following ingredient ratio, (wt%): 3,3'-diindolyhnethane 10-20; β-cyclodextrin 80-90, which are administered at a dosage of 200-400 mg of 3.3'-diindolyhnethane per day. The novel method for treating endometrial polyps makes it possible to avoid surgical intervention, to prevent recurrence of the disease, to avoid side effects and to reduce the risk of malignancy, through using a drug with enhanced bioavailability.

## Description

### Field of art

The invention relates to medicine and can be used in the treatment of benign hyperplastic processes in the endometrium.

### State of art

Endometrial polyp (polyp of the uterus body) is a benign mass associated with the growth of the endometrium (the inner mucous membrane of the uterus body) and is formed from its basal layer. Polyp of the uterus body is one of hyperplastic processes of the endometrium, which are manifested by thickening and changing the structure of the inner mucous membrane of the uterus.

Modern approaches to the treatment of endometrial polyps essentially limit to surgery and subsequent hormone therapy [Jayaprakasan K et al. Surgical intervention versus expectant management for endometrial polyps in subfertile women. Cochrane Database of Systematic Reviews 2014, Issue 8. Art. No.: CD009592]. So far, endometrial curettage, which entails many complications from development of endometriosis to malignant processes in the endometrium, is widely used. Numerous studies have proven that usual diagnostic curettage of an uterine cavity in 43-96% of cases does not provide complete removal of a polyp, as a result of which, within 1-2 months after curettage, a polyp that has not been completely removed is again identified [Litta P., Bartolucci C., Saccardi C., Codroma A.et al. Atypical endometrial lesions: hysteroscopic resection as an alternative to hysterectomy, Eur.J. Gynaecol. Oncol. 2013, N. 34(1): 51-3; Jiang et al. Do endometrial lesions require removal? A retrospective study BMC Women's Health, 2019, 19:61].

It is much less traumatic to remove polyps under visual control during hysteroscopy. Hysteroscopy with curettage of an uterine cavity also cannot be considered as a reliable and definitive method of treating endometrial polyps, since if untreated after six months in every second case, there is possible recurrence.

In the postoperative period, for the purpose of hormonal correction, following drugs are used: Dydrogesterone, Norethisterone, Medroxyprogesterone, Hydroxyprogesterone. Drug therapy with the use of gestagens does not give the desired result either, since the reasons for the development of high proliferative activity of endometrial cells are due not only to hormonal factors. In addition, long-term use of hormonal drugs leads to development of drug resistance and disease recurrence. In case of incomplete removal of a polyp, an order of subsequent hormonal therapy does not solve the problem, but only aggravates it [Adamyan L.V. et al. Combined benign tumors and hyperplastic processes of the uterus: a tutorial. - M.: Scientific Center for Obstetrics, Gynecology and Perinatology after V.I. Kulakova, 2015, 104].

In recent years, fundamentally new mechanisms for regulating excessive proliferative activity of endometrium have been described, which must be taken into account when developing new algorithms for treatment of this disease. In particular, there is a detailed description of the role of stem cells, where changes of epigenetic nature, leading to activation of cytocinesis, are found. [Gargett et al. Endometrial stem/progenitor cells: the first 10 years Human Reproduction Update 2016; Vol.22, No.2 pp. 137-163]. Increased activity of signaling pathways regulating intensity of endometrial cytocinesis is shown [Annu Makker et al. PI3K-Akt-mTOR and MAPK signaling pathways in polycystic ovarian syndrome, uterine leiomyomas and endometriosis: an update Gynecological Endocrinology, 2012; 28(3): 175-181]. In particular, the role of the phosphotidine-inositol signaling cascade in pathological processes in endometrium has been established. Importance of inflammatory processes in the endometrium, as the most important factor in initiation of hyperplastic diseases, has been investigated. From a molecular point of view, inflammation is accompanied by active synthesis of pro-inflammatory cytokines, which activate changes in the genetic program of cells, trigger processes of epithelial to mesenchymal transition and excessive synthesis of the nuclear transcription factor, which ultimately leads to hyperplastic processes, and in some cases, to malignant tissue degeneration [A. Steinbakk et al. Molecular biomarkers in endometrial hyperplasias predict cancer progression, Am. J. Obstet. Gynecol. 2011, Vol. 204 (4): 357].

An important role in the development of endometrial hyperplasia and pathologically associated carcinomas is given to inactivation (mutation or methylation of the gene, which leads to its silencing) of the PTEN (PHosphatase and TENsin) suppressor gene, which blocks a cell cycle. Normally, estrogens activate production of PTEN protein during the proliferative phase of the cycle. PTEN inactivation was noted in 63% of atypical hyperplasias and in 50-80% of endometrial adenocarcinomas [Camilla Nero et al. PTEN and Gynecological Cancers, Cancers 2019, 11, 1458; Yang et al. PTEN expression in benign human endometrial tissue and cancer in relation to endometrial cancer risk factors, Cancer Causes Control. 2015, 26(12): 1729-1736].

Earlier, we studied the possibility of treating hyperplastic processes of the endometrium with a drug based on DIM, dissolved in a mixture of fish oil and polysorbate. The method of treatment consisted in a patient taking a DIM solution containing as a carrier a mixture of fish oil and at least one polysorbate with the following content of components in wt%:

| | |
|---|---|
| 3,3'-diindolylmethane | 1-20 |
| fish oil | 10-20 |
| polysorbate | the rest, |

whereby the drug is taken in a dosage of 50 mg 2 times a day for 6 months. [RU 2419426 C1, publ. 05/27/2011].

However, a disadvantage of this approach was high frequency of side effects associated with development of diarrhea, probably due to the presence of polysorbate in the drug. This method is taken as a prototype.

### Essense of the invention

The technical problem solved by the invention is development of a new method for treatment of endometrial polyps, which allows avoiding surgery, disease recurrence, side effects and reducing the risk of malignancy and using a drug with increased bioavailability.

The technical problem is solved by a method of treating endometrial polyps, which consists in administering a drug containing 3,3'-diindolylmethane to a patient, while it is administered intravaginally at a dosage of 200-400 mg of 3,3'-diindolylmethane per day, in which case as a drug vaginal tablets are used containing a complex of 3,3'-diindolylmethane and β-cyclodextrin in the following ratio of components, mass %:

| | |
|---|---|
| 3,3'-diindolylmethane | 10-20 |
| β- cyclodextrin | 80-90, |

which are administered in a dosage of 200-400 mg of 3,3'-diindolylmethane per day.

### Theoretical basis of the invention

Diindolylmethane (DIM), its analogs and derivatives have a wide range of biological activities, which makes it possible to consider it as a very promising tool compound.

It has been experimentally proven that DIM has a very wide range of antiproliferative activity. Pronounced anti-tumor effect of DIM has been demonstrated in vitro and in vivo in models of tumors of the breast, ovaries, thyroid gland, intestines, and cervix [V. L. Maruthanila, J. Poornima, and S. Mirunalini, Attenuation of Carcinogenesis and the Mechanism Underlying by the Influence of Indole-3-carbinol and Its Metabolite 3,3'-Diindolylmethane: A Therapeutic Marvel, Advances in Pharmacological Sciences (1914); Feitelson et al. Sustained proliferation in cancer: mechanisms and novel therapeutic targets Semin Cancer Biol. 2015, 35(Suppl): 25-S54; Edward A. Ratovitski Anticancer Natural Compounds as Epigenetic Modulators of Gene Expression Current Genomics, 2017, 18: 175-205].

Recently discovered ability of DIM to exhibit anti-angiogenic activity is extremely important. Pathological vascular growth almost always accompanies hyper and neoplastic processes. It is known that without the formation of a network of capillary vessels supplying a newly formed tumor, which has reached a diameter of 1-2 mm, with oxygen and nutrients, its further growth is absolutely impossible. It was shown that in vitro micromolar concentrations of DIM effectively suppress proliferation and migration of endothelial cells, as well as their ability to form vessels. DIM administered subcutaneously to experimental animals (in vivo, 5 mg / kg, daily) inhibited pathological neoangiogenesis by 74%. It was found that the antiangiogenic activity of DIM is also due to its direct inhibitory effect on the HIF protein (hypoxia-induced factor) [Chang X, Tou JC, Hong C et al. 3,3'-Diindolylmethane inhibits angiogenesis and the growth of transplantable human breast carcinoma in athymic mice, Carcinogenesis 2005, 264(4), 771-778; McCarty MF, Block KI. Multifocal angiostatic therapy: an update, Integrative Cancer Therapies 2005, 4(4): 301-314].

The most important molecular target, to block activity of which, action of developed and introduced into clinical practice modern targeted drugs is directed, is the nuclear transcription factor NF-κB. It has been proven that this factor mediates inflammatory response and also plays an important role in the regulation of proliferative (anti-apoptotic), angiogenic, migratory and invasive cellular activities, carrying out the final stage of signaling cascades induced by growth factors and cytokines. In this case, a key point is translocation of the active factor into a nucleus and activation of transcription of genes responsible for these processes. It was found that in vitro DIMs effectively suppress nuclear translocation and factor activity NF-κB [Rahman KM, Ali S, Aboukameel A et al. Inactivation of NF-kappaB by 3,3'-diindolylmethane contributes to increased apoptosis induced by chemotherapeutic agent in breast cancer cells, Mol. Cancer Ther 2007, 6(10): 2757-2765; Rahman KM, Sarkar FH. Inhibition of nuclear translocation of Nuclear Factor-κB contributes to 3,3'-diindolylmethane-induced apoptosis in breast cancer cells, Cancer Res. 2005, 65: 364-371]. This means that in addition to the antiproliferative and antiangiogenic effects, the drug produced on basis of DIM is capable of suppressing local inflammatory reactions that often accompany hyper- and neoplastic processes in hormone-dependent organs and tissues.

As mentioned before, in endometrial hyperplasia, there is an increased activity of signaling cascades that stimulate the proliferation of endometrial cells. Particular importance is attached to PI3K/Akt/mTOR/NF-κB signaling pathway, which is inhibited by DIM, which can be of great practical importance in the treatment of proliferative endometrial pathologies with drugs based on indole derivatives. An important property of DIM is its ability to inhibit processes of epithelial to mesenchymal transition (EMT), which significantly increases the risk of malignancy [Popolo A. et al. Two likely targets for the anti-cancer effect of indole derivatives from cruciferous vegetables: PI3K/Akt/mTOR signaling pathway and the aryl hydrocarbon receptor Seminars in Cancer Biology 42017; 46:132-137; Ahmad et al. Targeted Regulation of I3K/Akt/mTOR/NF-κB Signaling by Indole Compounds and their Derivatives: Mechanistic Details and Biological Implications for Cancer Therapy Anticancer Agents Med Chem. 2013; 13(7): 1002-1013; Eva Colas et al. The EMT signaling pathways in endometrial carcinoma Clin Transl Oncol (2012) 14:715-720; Andrea P. Myers, New Strategies in Endometrial Cancer: Targeting the PI3K/mTOR Pathway-The Devil Is in the Details Clin Cancer Res 2013; 19(19); 5264-74].

Oral dosing of DIM is the most common. However, the oral bioavailability of DIM is severely limited due to its very low solubility and low absorption efficacy in the small intestine. DIM usually exhibits low solubility in body fluids and has a very limited ability to penetrate barrier membranes, which does not allow reaching the required concentrations of the active substance in the lesions.

As mentioned before, the disadvantage of the method for treating hyperplastic processes of endometrium with a drug based on DIM dissolved in a mixture of fish oil and polysorbate according to the prototype is high frequency of side effects associated with development of diarrhea, probably due to presence of polysorbate in the drug.

We have previously developed a drug in the form of vaginal suppositories containing diindolylmethane [RU 2419426 C1, publ. 05/27/2011]. Clinical testing of this solution has shown high efficacy in treatment of cervical neoplasias [Ashrafian et al. Double-blind randomized placebocontrolled multicenter clinical trial (phase IIa) on diindolylmethane's efficacy and safety in the treatment of CIN: implications for cervical cancer prevention The EPMA Journal (2015) 6:25]. Administrated vaginally, there was no significant DIM penetration into the systemic blood, however, DIM accumulated in endometrium at therapeutic concentrations. However, vaginal administration of the drug in the form of suppositories or gel (cream) is accompanied by a burning and discomfort sensations, often forcing patients to interrupt the course of treatment. Considering this, as well as the aforementioned molecular mechanisms of DIM action, namely antiestrogenic activity, restoration of apoptosis processes, antiproliferative, antitumor and antiangiogenic effects, inhibition of EMF and epigenetic activity, an objective of this invention was to develop a method for treatment of endometrial polyps using intravaginal drugs containing diindolylmethane in the form of vaginal tablets.

### Examples of the invention

### Example 1.

Preparation of a dosage formulation 3,3'-diindolylmethane and β-cyclodextrin (DIM + βCD).

β-cyclodextrin powder in an amount of 5600 g (moisture content 12%) was dissolved in 22.6 liters of water, gradually stirring. 713 g of 3,3'-diindolylmethane was added to the β-cyclodextrin solution. The suspension was stirred for 4 hours. The resulting suspension was fed into a spray dryer at a gas temperature of 1800C. The yield of the obtained complex 3,3'-diindolylmethane - β-cyclodextrin (DIM + βCD) was 5350 g. The dry product was sieved through a sieve with a riddling element opening of 1 m. The ratio of DIM and dry β-cyclodextrin in the resulting product was 12.6 wt% and 87.4 wt%, respectively, which corresponds to the molar ratio of these components in the complex.

The resulting complex dissolves completely in water.

A solution of the DIM + βCD complex was subjected to chromatographic analysis. The results of physicochemical analysis showed almost complete coincidence of the chromatograms of pure 3,3'-diindolimethane and the DIM + βCD complex.

The complex was also prepared with the following ratios of DIM and dry β-cyclodextrin.
1. DIM 10 mass %, βCD 90 mass %.
2. DIM 20 mass %, βCD 80 mass %.

The deviation of the ratio from the molar ratio within the specified limits and a slight excess of one or another component in the resulting product does not affect the properties of the drug in general.

### Example 2.

### Study of the concentration of 3,3'-diindolimethane in the blood plasma of patients/

Clinical studies have compared the efficacy of the water-soluble DIM + βCD complex and crystalline 3,3'-diindolylmethane in equal dosages when taken orally. In all studied groups of patients, there was a pronounced positive response when taking the DIM + βCD complex and the absence of such when taking crystalline 3,3'-diindolylmethane. The results of measuring concentration of 3,3'-diindolylmethane in the plasma of patients are presented in Table 1.

**Table 1**

| Drug | Dosage, mg | | Plasma DIM concentration, ng/ml | |
|---|---|---|---|---|
| Crystal DIM | | 10 | | not determined |
| DIM + βCD | | 10 | | more than 100 |
| Crystal DIM | | 100 | | less than 30 |
| DIM + βCD | | 100 | | more than 300 |

The test results showed that stable bioavailability parameters were achieved using the water-soluble DIM + βCD complex at a dosage of 100 mg per day. At the same time, the concentration of DIM in plasma when administrating 100 mg of water-soluble complexes was more than 300 ng/ml.

### Example 3.

Formulations of vaginal tablets
Formulation 1. DIM 10 mass parts,
β-cyclodextrin 90 mass parts.
Magnesium stearate 1 mass part.
Formulation 2. DIM 20 mass parts,
β-cyclodextrin 80 mass parts.
Magnesium stearate 1 mass part.

The tablets may be coated to facilitate insertion into the vagina.

Vaginal tablets can be made with other known excipients or can be obtained by simple compression of the substance without the use of excipients.

### Option 4.

Clinical studies on the efficacy of therapy for endometrial polyps with intravaginal drugs containing diindolylmethane

15 women were selected in the age range of 18-40 years with a diagnosis of endometrial polyps, confirmed by ultrasound examination of the uterine cavity and diagnostic hysteroscopy, for clinical studies. The women were divided into 3 groups of 5 people each. Each group was prescribed therapy with intravaginal drugs containing diindolylmethane. The first group received vaginal suppositories containing DIM 100mg, one suppository in the morning and in the evening for 90 days. Group 2 received DIM vaginal tablets (2 people at 200 mg, 1 person 300 mg, 2 people at 400 mg) at night for 90 days, half of group 2 - vaginal tablets of formulation No. 1, the other half of group 2 - vaginal tablets of formulation No. 2. Group 3 was prescribed a vaginal gel or cream, which was injected into the vagina with a special syringe at night. The dose in terms of DIM was 200-300 mg. Duration of treatment was 90 days.

After the course of therapy, all the women under study were followed-up. On the top of already administered therapy, the clinical manifestations decreased: pain in the lower abdomen and spotting bleeding. Control ultrasound and hysteroscopy showed no pathological changes in the endometrium. Observation of the patients for a year did not show recurrence of the disease. It should be noted that the use of suppositories and gel (cream) was accompanied by a burning and discomfort sensations, often forcing patients to interrupt the course of treatment. This observation is probably due to the rapid release of the active substance. The therapeutic efficacy of the suppositories and the gel was also lower than in the group of patients taking the pills. In addition, both suppositories and the gel required a special storage regime (+ 4 C°), since at room temperature the diindolylmethane contained in them was oxidized, which led to a color change (a pink tint appeared) and the concentration of the active substance decreased. Whereas tablets with DIM with cyclodextrin were stored at room temperature without discoloration and loss of concentration of the active substance.

## Claims

1. A method of treating endometrial polyps, which consists in administering a drug containing 3,3'-diindolylmethane to a patient, **characterized by** that as the drug vaginal tablets are used containing a complex of 3,3'-diindolylmethane and β-cyclodextrin in the following ratio of components, mass %:
| | |
|---|---|
| 3,3'-diindolylmethane | 10-20 |
| β- cyclodextrin | 80-90, |
which are administered in a dosage of 200-400 mg of 3,3'-diindolylmethane per day.
